(19) Europäisches Patentamt

European Patent Office

Office européen des brevets

(11) EP 4 506 948 A1

(12) EUROPEAN PATENT APPLICATION
published in accordance with Art. 153(4) EPC

(43) Date of publication:
12.02.2025 Bulletin 2025/07

(51) International Patent Classification (IPC):
G16B 40/20 (2019.01)  C07K 16/00 (2006.01)
C12N 15/13 (2006.01)  G01N 33/15 (2006.01)
G16H 20/10 (2018.01)

(21) Application number: 23803291.6

(22) Date of filing: 05.04.2023

(52) Cooperative Patent Classification (CPC):
C07K 16/00; G01N 33/15; G16B 40/20;
G16H 20/10

(86) International application number:
PCT/JP2023/014063

(87) International publication number:
WO 2023/218808 (16.11.2023 Gazette 2023/46)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 09.05.2022 JP 2022077239

(71) Applicant: FUJIFILM Corporation
Tokyo 106-8620 (JP)

(72) Inventor: WANG, Caihua
Ashigarakami-gun, Kanagawa 258-8577 (JP)

(74) Representative: Hoffmann Eitle
Patent- und Rechtsanwälte PartmbB
Arabellastraße 30
81925 München (DE)

(54) **PHARMACEUTICAL PRODUCTION ASSISTANCE DEVICE, METHOD OF OPERATING PHARMACEUTICAL PRODUCTION ASSISTANCE DEVICE, AND PROGRAM FOR OPERATING PHARMACEUTICAL PRODUCTION ASSISTANCE DEVICE**

(57) A pharmaceutical support device includes a processor, in which the processor is configured to: acquire prescription information related to a prescription of a candidate preservation solution that is a candidate for a preservation solution for a biopharmaceutical containing a protein; acquire a plurality of types of measurement data actually measured in a test for confirming preservation stability of the candidate preservation solution; use a machine learning model that outputs prediction data at a future time point of first measurement data, which is at least one type of the plurality of types of measurement data; and input the prescription information, the first measurement data, and at least one type of second measurement data other than the first measurement data among the plurality of types of measurement data to the machine learning model and output the prediction data from the machine learning model.

FIG. 9

EP 4 506 948 A1

**Description**

BACKGROUND OF THE INVENTION

1. Field of the Invention

The technology of the present disclosure relates to a pharmaceutical support device, an operation method of a pharmaceutical support device, and an operation program of a pharmaceutical support device.

2. Description of the Related Art

In recent years, biopharmaceuticals have attracted attention due to high drug efficacy and low side effects. The biopharmaceutical has, for example, a protein, such as interferon or an antibody, as an active ingredient. The biopharmaceutical is preserved in a preservation solution. It is important to prescribe a preservation solution (also referred to as formulation prescription) suitable for the biopharmaceutical in order to stably maintain the quality of the biopharmaceutical.

The preservation solution includes a buffer solution, an additive, and a surfactant. The prescription of the preservation solution is, for example, the type and concentration of each of the buffer solution, the additive, and the surfactant and a hydrogen ion exponent (potential of hydrogen (pH) value) of the preservation solution.

In the related art, in a case where the prescription of the preservation solution is determined, a plurality of types of preservation solutions are prepared, for example, while changing a combination of the buffer solution, the additive, and the surfactant, and the preservation stability of the protein in each preservation solution is confirmed by an actual test. However, it takes a lot of time and effort to perform this operation. Therefore, for example, as in <Theresa K. Cloutier, etc., Machine Learning Models of Antibody-Excipient Preferential Interactions for Use in Computational Formulation Design, Mol. Pharmaceutics, 17, 9, 3589-3599, 2020.> (hereinafter, referred to as Non-Patent Document), a technology has been proposed that predicts preservation stability of a protein with respect to an additive based on information of a protein in a biopharmaceutical and information of an additive in the preservation solution, using a molecular dynamics (MD) method or a machine learning model, without performing any test.

SUMMARY OF THE INVENTION

In the technology disclosed in Non-Patent Document, the preservation stability of the preservation solution is not confirmed by the actual test. Therefore, there is a risk that a prediction result that is far from the original preservation stability may be obtained. Therefore, the present inventors are considering preparing a plurality of types of preservation solutions, performing a test for, for example, two weeks, and predicting the preservation stability of the protein in each preservation solution after, for example, four weeks using the measurement data actually measured at that time point.

However, for the preservation stability of the preservation solution, there are various types of preservation stability, such as preservation stability of the protein with respect to aggregation and preservation stability of the protein with respect to temperature, including the preservation stability of the protein with respect to the additive described in Non-Patent Document. Further, these plurality of types of preservation stability are correlated with each other. Therefore, for example, in a case of predicting measurement data for confirming preservation stability of the protein with respect to aggregation, there is a concern that the prediction accuracy cannot be improved by using only the measurement data for confirming the preservation stability of the protein with respect to the aggregation obtained at that time point.

An embodiment according to the technology of the present disclosure provides a pharmaceutical support device, an operation method of a pharmaceutical support device, and an operation program of a pharmaceutical support device that can improve prediction accuracy of preservation stability of a preservation solution.

According to the present disclosure, there is provided a pharmaceutical support device comprising a processor in which the processor is configured to: acquire prescription information related to a prescription of a candidate preservation solution that is a candidate for a preservation solution for a biopharmaceutical containing a protein; acquire a plurality of types of measurement data actually measured in a test for confirming preservation stability of the candidate preservation solution; use a machine learning model that outputs prediction data at a future time point of first measurement data, which is at least one type of the plurality of types of measurement data; and input the prescription information, the first measurement data, and at least one type of second measurement data other than the first measurement data among the plurality of types of measurement data to the machine learning model and output the prediction data from the machine learning model.

It is preferable that the measurement data is time-series data measured at least at two time points.

It is preferable that the prescription information includes at least one of a type of each of a buffer solution, an additive, or a surfactant contained in the candidate preservation solution, a concentration of each of the buffer solution, the additive, or the surfactant, or a hydrogen ion exponent of the candidate preservation solution.

It is preferable that the measurement data includes data for confirming preservation stability of the protein with respect to aggregation and data for confirming preservation stability of the protein with respect to a temperature.

It is preferable that the measurement data is any of aggregation analysis data of a sub-visible particle of the protein in the candidate preservation solution, analysis data of the protein in the candidate preservation solution by a dynamic light scattering method, analysis data of the protein in the candidate preservation solution by size exclusion chromatography, or analysis data of the protein in the candidate preservation solution by differential scanning calorimetry.

It is preferable that the machine learning model outputs the prediction data of one type of the first measurement data.

It is preferable that the machine learning model outputs the prediction data of two or more types of the first measurement data.

It is preferable that the processor is configured to also input a feature amount derived based on protein information related to the protein to the machine learning model.

It is preferable that the protein is an antibody.

According to the present disclosure, there is provided an operation method of a pharmaceutical support device, including: acquiring prescription information related to a prescription of a candidate preservation solution that is a candidate for a preservation solution for a biopharmaceutical containing a protein; acquiring a plurality of types of measurement data actually measured in a test for confirming preservation stability of the candidate preservation solution; using a machine learning model that outputs prediction data at a future time point of first measurement data, which is at least one type of the plurality of types of measurement data; and inputting the prescription information, the first measurement data, and at least one type of second measurement data other than the first measurement data among the plurality of types of measurement data to the machine learning model and outputting the prediction data from the machine learning model.

According to the present disclosure, there is provided an operation program of a pharmaceutical support device causing a computer to execute a process including: acquiring prescription information related to a prescription of a candidate preservation solution that is a candidate for a preservation solution for a biopharmaceutical containing a protein; acquiring a plurality of types of measurement data actually measured in a test for confirming preservation stability of the candidate preservation solution; using a machine learning model that outputs prediction data at a future time point of first measurement data, which is at least one type of the plurality of types of measurement data; and inputting the prescription information, the first measurement data, and at least one type of second measurement data other than the first measurement data among the plurality of types of measurement data to the machine learning model and outputting the prediction data from the machine learning model.

According to the technology of the present disclosure, it is possible to provide a pharmaceutical support device, an operation method of a pharmaceutical support device, and an operation program of a pharmaceutical support device that can improve prediction accuracy of preservation stability of a preservation solution.

BRIEF DESCRIPTION OF THE DRAWINGS

Fig. 1 is a diagram illustrating a pharmaceutical support server and an operator terminal.
Fig. 2 is a diagram illustrating a series of flows of preparation of a candidate preservation solution, addition of an antibody, a stress test, and measurement of measurement data, and prescription information.
Fig. 3 is a diagram illustrating measurement data of the stress test in the 0th week.
Fig. 4 is a diagram illustrating measurement data of the stress test in the first week.
Fig. 5 is a diagram illustrating measurement data of the stress test in the second week.
Fig. 6 is a block diagram illustrating a computer constituting the pharmaceutical support server.
Fig. 7 is a block diagram illustrating processing units of a CPU of the pharmaceutical support server.
Fig. 8 is a diagram illustrating a preservation stability prediction model.
Fig. 9 is a diagram illustrating an outline of a prediction using an SVP aggregation analysis data prediction model.
Fig. 10 is a table showing learning data of the SVP aggregation analysis data prediction model.
Fig. 11 is a diagram illustrating an outline of a process in a learning phase of the SVP aggregation analysis data prediction model.
Fig. 12 is a diagram illustrating an outline of a prediction using a DLS analysis data prediction model.
Fig. 13 is a diagram illustrating an outline of a prediction using an SEC analysis data prediction model.
Fig. 14 is a flowchart illustrating a processing procedure of the pharmaceutical support server.
Fig. 15 is a diagram illustrating another example of the prescription information.
Fig. 16 is a diagram illustrating a second embodiment in which prediction data of SVP aggregation analysis data, DLS analysis data, and SEC analysis data are output from one preservation stability prediction model.
Fig. 17 is a diagram illustrating an outline of a process in a learning phase of the preservation stability prediction model illustrated in Fig. 16.
Fig. 18 is a diagram illustrating a feature amount derivation unit that derives a feature amount based on antibody information.
Fig. 19 is a diagram illustrating a third embodiment in which the feature amount is also input to the preservation stability prediction model.

DESCRIPTION OF THE PREFERRED EMBODIMENTS

[First Embodiment]

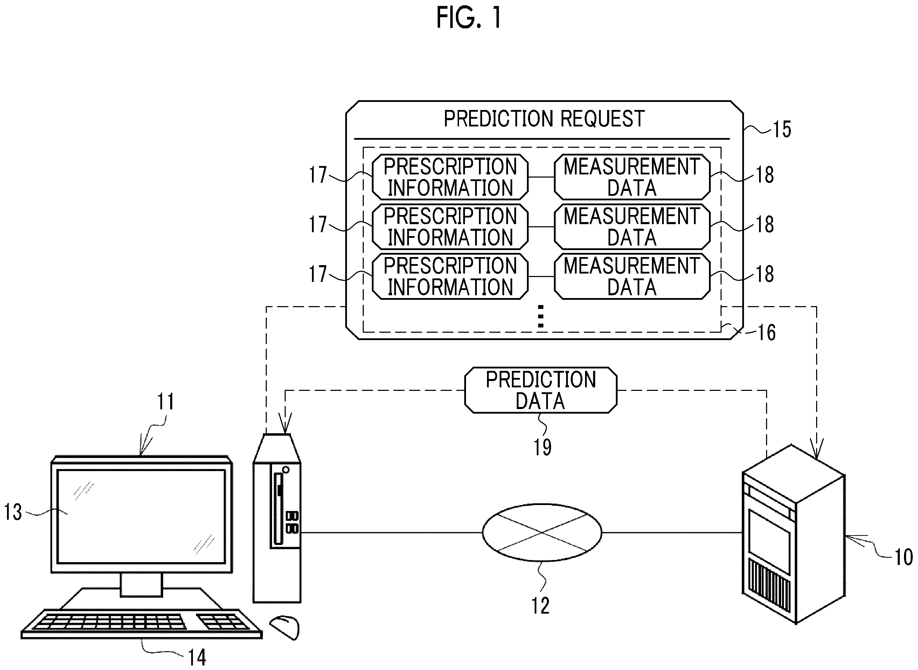

As illustrated in Fig. 1 as an example, a pharmaceutical support server 10 is connected to an operator terminal 11 through a network 12. The pharmaceutical support server 10 is an example of a "pharmaceutical support device" according to the technology of the present disclosure. The operator terminal 11 is installed, for example, in a pharmaceutical facility and is operated by an operator, such as a pharmacist, who is involved in the production of biopharmaceuticals in the pharmaceutical facility. The operator terminal 11 has a display 13 and an input device 14 such as a keyboard and a mouse. The network 12 is, for example, a wide area network (WAN) such as the Internet or a public communication network. In addition, in Fig. 1, only one operator terminal 11 is connected to the pharmaceutical support server 10. However, in practice, a plurality of operator terminals 11 in a plurality of pharmaceutical facilities are connected to the pharmaceutical support server 10.

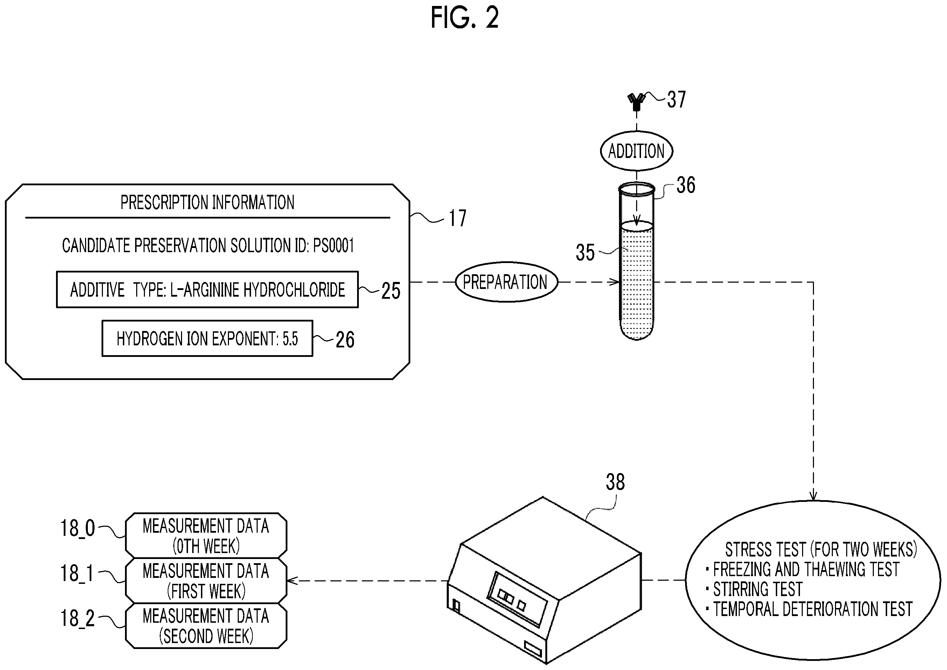

The operator terminal 11 transmits a prediction request 15 to the pharmaceutical support server 10. The prediction request 15 is a request for the pharmaceutical support server 10 to predict preservation stability of a candidate preservation solution 35 (see Fig. 2), which is a candidate for a preservation solution for the biopharmaceutical. The prediction request 15 includes a prescription information and measurement data set group 16. The prescription information and measurement data set group 16 includes a plurality of sets of prescription information 17 and measurement data 18. The set of the prescription information 17 and the measurement data 18 is registered for each of a plurality of types, for example, several to several tens of types of candidate preservation solutions 35. The prescription information 17 is information related to the prescription of the candidate preservation solution 35. The measurement data 18 is data indicating the preservation stability of the candidate preservation solution 35 actually measured by an actual test. The operator operates the input device 14 to input the prescription information 17 and the measurement data 18. In addition, although not illustrated, the prediction request 15 also includes terminal identification data (ID) for uniquely identifying the operator terminal 11 which is a transmission source of the prediction request 15.

In a case where the prediction request 15 is received, the pharmaceutical support server 10 derives the prediction data 19 at a future time point of the measurement data 18. The pharmaceutical support server 10 delivers the prediction data 19 to the operator terminal 11 which is the transmission source of the prediction request 15. In a case where the prediction data 19 is received, the operator terminal 11 displays the prediction data 19 on the display 13 such that the prediction data 19 is provided to be viewed by the operator.

As illustrated in Fig. 2 as an example, the prescription information 17 includes a type 25 of additive contained in the candidate preservation solution 35 and a hydrogen ion exponent 26 of the candidate preservation solution 35. A candidate preservation solution ID for uniquely identifying the candidate preservation solution 35 is given to the prescription information 17. The operator creates the prescription information 17, relying on his/her own experience, and the like. For example, the type 25 of the additive is digitized as follows: an additive A is digitized as "1"; and an additive B is digitized as "2".

The operator actually prepares the candidate preservation solution 35 in a test tube 36 in accordance with the prescription information 17. Then, the antibody 37 is added to the prepared candidate preservation solution 35. Thereafter, a stress test including a freezing and thawing test of repeating freezing and thawing of the candidate preservation solution 35 to which the antibody 37 has been added a plurality of times, for example, three times, a stirring test of stirring the candidate preservation solution 35 to which the antibody 37 has been added with a stirrer, and a temporal deterioration test of leaving the candidate preservation solution 35 to which the antibody 37 has been added as it is is performed for two weeks. During the stress test for two weeks, the measurement data 18 is actually measured using a measurement device 38. The antibody 37 is an example of a "protein" according to the technology of the present disclosure.

Fig. 2 illustrates an example in which the measurement data (denoted as measurement data (0th week) in the drawing) 18_0 in the 0th week of the stress test, the measurement data (denoted as measurement data (first week) in the drawing) 18_1 in the first week of the stress test, and the measurement data (denoted as measurement data (second week) in the drawing) 18_2 in the second week of the stress test are measured, respectively. Here, the 0th week of the stress test is the start of the stress test. In addition, the first week of the stress test is a time point of one week after the start of the stress test, and the second week of the stress test is a time point of two weeks after the start of the stress test. The measurement device 38 transmits the measurement data 18_0 to 18_2 to the operator terminal 11.

The freezing and thawing test and the stirring test are performed in the 0th week of the stress test. On the other hand, the temporal deterioration test is continuously performed in the 0th to second week of the stress test. The operator prepares each of the candidate preservation solution 35 for the freezing and thawing test, the stirring test, and for the temporal deterioration test and adds the antibody 37 to the prepared candidate preservation solution 35.

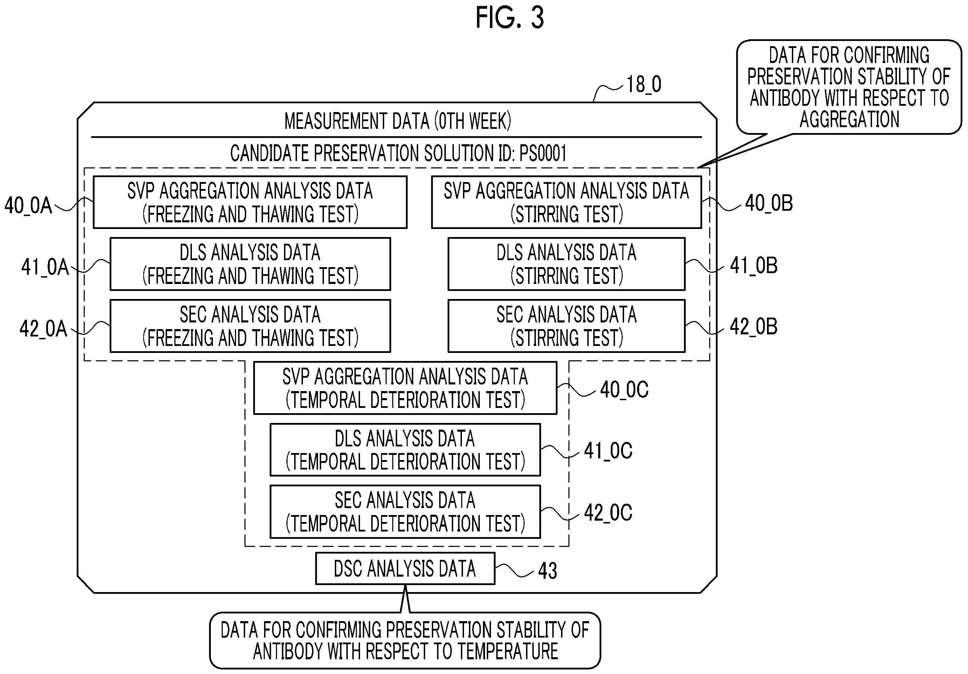

As illustrated in Figs. 3 to 5 as an example, the measurement data 18 includes aggregation analysis data (hereinafter, referred to as SVP aggregation analysis data) 40 of sub-visible particle (SVP) of the antibody 37 in the candidate preservation solution 35 and analysis data (hereinafter, referred to as DLS analysis data) 41 of the antibody 37 in the candidate preservation solution 35 by a dynamic light scattering (DLS) method. The SVP aggregation analysis data 40 indicates the amount of SVPs of the antibody 37 in the candidate preservation solution 35 that causes a reduction in the drug efficacy of the biopharmaceutical. Similarly, the DLS analysis data 41 indicates the amount of nanoparticles of the antibody 37 in the candidate preservation solution 35 that causes the reduction in the drug efficacy of the biopharmaceutical.

In addition, the measurement data 18 includes analysis data (hereinafter, referred to as SEC analysis data) 42 of the antibody 37 in the candidate preservation solution 35 by size exclusion chromatography (SEC). The SEC analysis data 42 indicates a molecular weight distribution of the antibody 37 in the candidate preservation solution 35. The SVP aggregation analysis data 40, the DLS analysis data 41, and the SEC analysis data 42 are data for confirming preservation stability of the antibody 37 with respect to aggregation. That is, the SVP aggregation analysis data 40, the DLS analysis data 41, and the SEC analysis data 42 are examples of "data for confirming preservation stability of a protein with respect to aggregation" according to the technology of the present disclosure. A candidate preservation solution ID for uniquely identifying the candidate preservation solution 35 is given to the measurement data 18, as in the prescription information 17.

In Fig. 3, the measurement data 18_0 includes the SVP aggregation analysis data (denoted as SVP aggregation analysis data (freezing and thawing test) in the drawing) 40_0A, the DLS analysis data (denoted as DLS analysis data (freezing and thawing test) in the drawing) 41_0A, and the SEC analysis data (denoted as SEC analysis data (freezing and thawing test) in the drawing) 42_0A, which are measured after the freezing and thawing test. In addition, the measurement data 18_0 includes the SVP aggregation analysis data (denoted as SVP aggregation analysis data (stirring test) in the drawing) 40_0B, the DLS analysis data (denoted as DLS analysis data (stirring test) in the drawing) 41_0B, and the SEC analysis data (denoted as SEC analysis data (stirring test) in the drawing) 42_0B, which are measured after the stirring test. Further, the measurement data 18_0 includes the SVP aggregation analysis data (denoted as SVP aggregation analysis data (temporal deterioration test) in the drawing) 40_0C, DLS analysis data (denoted as DLS analysis data (temporal deterioration test) in the drawing) 41_0C, and SEC analysis data (denoted as SEC analysis data (temporal deterioration test) in the drawing) 42_0C of the temporal deterioration test.

The measurement data 18_0 includes analysis data (hereinafter, referred to as DSC analysis data) 43 of the antibody 37 in the candidate preservation solution 35 by differential scanning calorimetry (DSC) in addition to the SVP aggregation analysis data 40, the DLS analysis data 41, and the SEC analysis data 42. The DSC analysis data 43 indicates values related to thermophysical property values such as the glass transition point and crystallization temperature of the antibody 37 in the candidate preservation solution 35. The DSC analysis data 43 is data for confirming preservation stability of the antibody 37 with respect to a temperature. That is, the DSC analysis data 43 is an example of "data for confirming preservation stability of a protein with respect to a temperature" according to the technology of the present disclosure.

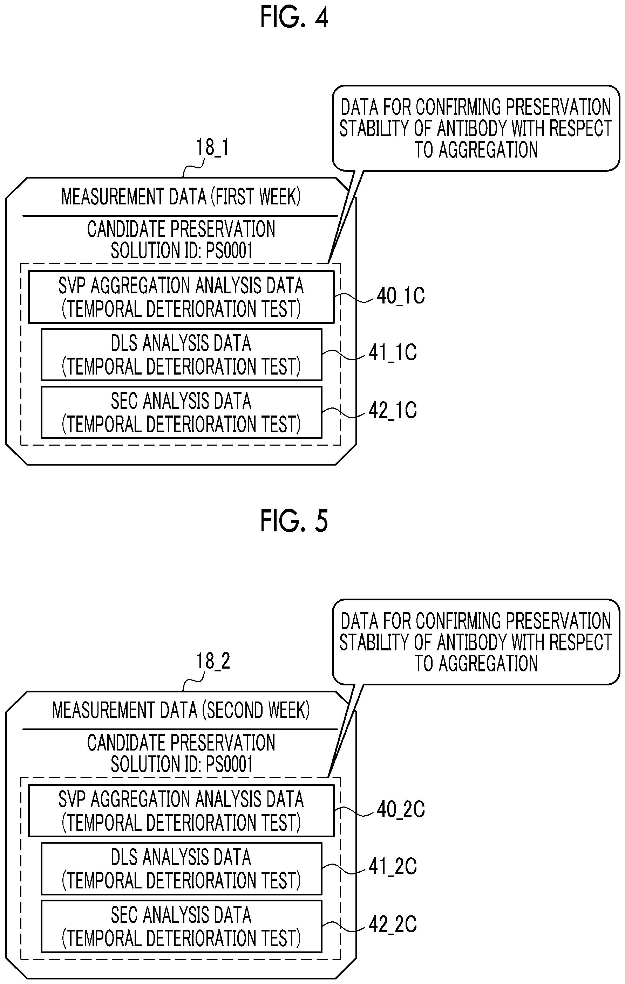

In Fig. 4, the measurement data 18_1 includes the SVP aggregation analysis data 40_1C, the DLS analysis data 41_1C, and the SEC analysis data 42_1C of the temporal deterioration test. In addition, in Fig. 5, the measurement data 18_2 includes the SVP aggregation analysis data 40_2C, the DLS analysis data 41_2C, and the SEC analysis data 42_2C of the temporal deterioration test. The measurement data 18_1 and 18_2 do not include the DSC analysis data 43, unlike the measurement data 18_0.

As described above, the measurement data 18 includes a plurality of types of measurement data such as the SVP aggregation analysis data 40, the DLS analysis data 41, the SEC analysis data 42, and the DSC analysis data 43. In addition, the measurement data 18 includes the measurement data 18_0 in the 0th week, the measurement data 18_1 in the first week, and the measurement data 18_2 in the second week of the stress test. Therefore, the measurement data 18 is time-series data measured at least at two time points.

Further, the measurement data 18 includes data measured after the freezing and thawing test, such as the SVP aggregation analysis data 40_0A, data measured after the stirring test, such as the DLS analysis data 41_0B, and data measured for the temporal deterioration test, such as the SVP aggregation analysis data 40_1C. As described above, it is preferable that the measurement data 18 includes data obtained from tests different from each other.

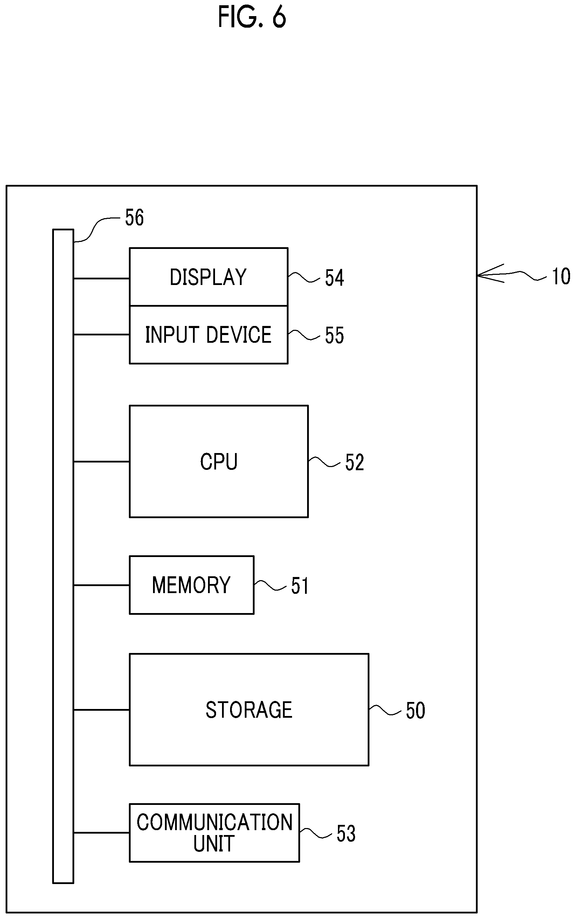

As illustrated in Fig. 6 as an example, a computer constituting the pharmaceutical support server 10 comprises a storage 50, a memory 51, a central processing unit (CPU) 52, a communication unit 53, a display 54, and an input device 55. These units are connected to each other through a bus line 56.

The storage 50 is a hard disk drive that is provided in the computer constituting the pharmaceutical support server 10 or that is connected to the computer through a cable or a network. Alternatively, the storage 50 is a disk array in which a plurality of hard disk drives are connected. The storage 50 stores, for example, a control program, such as an operating system, various application programs, and various types of data associated with these programs. In addition, a solid state drive may be used instead of the hard disk drive.

The memory 51 is a work memory used by the CPU 52 to execute processes. The CPU 52 loads the program stored in the storage 50 to the memory 51 and executes a process corresponding to the program. Therefore, the CPU 52 controls each unit of the computer comprehensively. The CPU 52 is an example of a "processor" according to the technology of the present disclosure. In addition, the memory 51 may be provided in the CPU 52.

The communication unit 53 controls the transmission of various types of information to an external device such as the operator terminal 11. The display 54 displays various screens. The various screens have operation functions by a graphical user interface (GUI). The computer constituting the pharmaceutical support server 10 receives an input of an operation instruction from the input device 55 through the various screens. The input device 55 is, for example, a keyboard, a mouse, a touch panel, and a microphone for voice input.

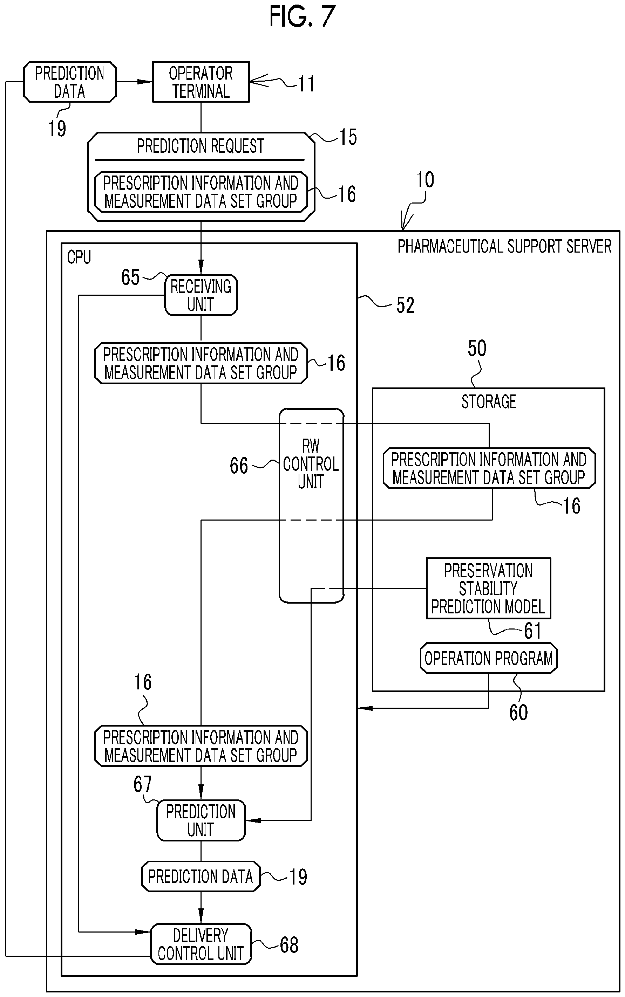

As illustrated in Fig. 7 as an example, an operation program 60 is stored in the storage 50 of the pharmaceutical support server 10. The operation program 60 is an application program for causing the computer to function as the pharmaceutical support server 10. That is, the operation program 60 is an example of "an operation program of a pharmaceutical support device" according to the technology of the present disclosure. The storage 50 also stores a preservation stability prediction model 61.

In a case where the operation program 60 is started, the CPU 52 of the computer constituting the pharmaceutical support server 10 functions as a receiving unit 65, a read and write (hereinafter, abbreviated to RW) control unit 66, a prediction unit 67, and a delivery control unit 68 in cooperation with the memory 51 and the like.

The receiving unit 65 receives the prediction request 15 from the operator terminal 11. As described above, the prediction request 15 includes the prescription information and measurement data set group 16, and the prescription information and measurement data set group 16 includes the prescription information 17 and the measurement data 18. Therefore, the receiving unit 65 acquires the prescription information 17 and the measurement data 18 by receiving the prediction request 15. The receiving unit 65 outputs the prescription information and measurement data set group 16 to the RW control unit 66. In addition, the receiving unit 65 outputs the terminal ID of the operator terminal 11 (not illustrated) to the delivery control unit 68.

The RW control unit 66 controls the storage of various types of data in the storage 50 and the reading-out of various types of data in the storage 50. For example, the RW control unit 66 stores the prescription information and measurement data set group 16 from the receiving unit 65 in the storage 50. Further, the RW control unit 66 reads out the prescription information and measurement data set group 16 from the storage 50 and outputs the prescription information and measurement data set group 16 to the prediction unit 67.

Furthermore, the RW control unit 66 reads out the preservation stability prediction model 61 from the storage 50 and outputs the preservation stability prediction model 61 to the prediction unit 67.

The prediction unit 67 derives the prediction data 19 from the prescription information 17 and the measurement data 18 using the preservation stability prediction model 61. The prediction unit 67 outputs the derived prediction data 19 to the delivery control unit 68.

The delivery control unit 68 performs control to deliver the prediction data 19 to the operator terminal 11 which is the transmission source of the prediction request 15. In this case, the delivery control unit 68 specifies the operator terminal 11, which is the transmission source of the prediction request 15, based on the terminal ID from the receiving unit 65.

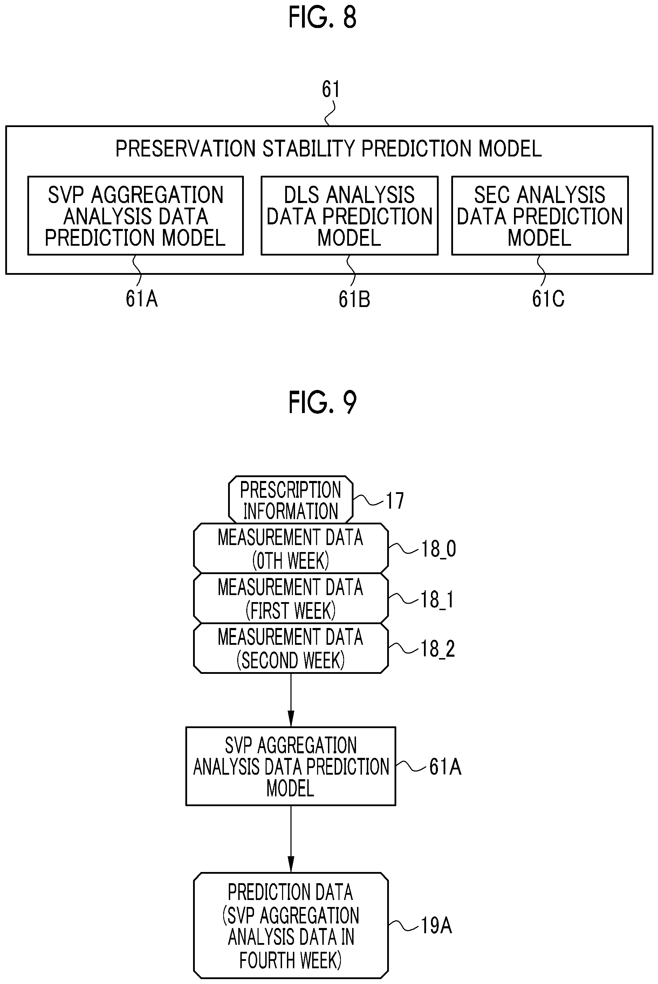

As illustrated in Fig. 8 as an example, the preservation stability prediction model 61 is composed of an SVP aggregation analysis data prediction model 61A, a DLS analysis data prediction model 61B, and an SEC analysis data prediction model 61C. The SVP aggregation analysis data prediction model 61A, the DLS analysis data prediction model 61B, and the SEC analysis data prediction model 61C are constructed by, for example, a neural network or a support vector regression (SVR) algorithm. The SVP aggregation analysis data prediction model 61A, the DLS analysis data prediction model 61B, and the SEC analysis data prediction model 61C are examples of a "machine learning model" according to the technology of the present disclosure.

As illustrated in Fig. 9 as an example, the prediction unit 67 inputs a set of the prescription information 17 and the measurement data 18_0 to 18_2 of one candidate preservation solution 35 to the SVP aggregation analysis data prediction model 61A. Then, the prediction data 19A is output from the SVP aggregation analysis data prediction model 61A. The prediction data 19A is data in which the SVP aggregation analysis data 40 in the fourth week of the stress test is predicted. In this case, the SVP aggregation analysis data 40 is an example of "first measurement data" according to the technology of the present disclosure. As described above, the measurement data 18 includes the SVP aggregation analysis data 40, the DLS analysis data 41, the SEC analysis data 42, and the DSC analysis data 43. Therefore, in this case, the DLS analysis data 41, the SEC analysis data 42, and the DSC analysis data 43 other than the SVP aggregation analysis data 40 are examples of "second measurement data" according to the technology of the present disclosure.

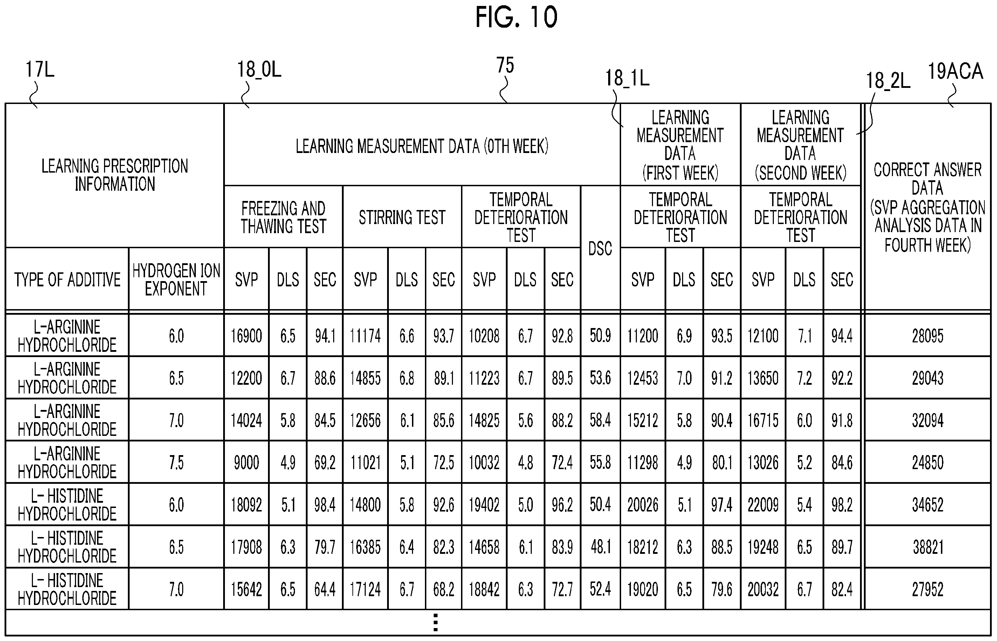

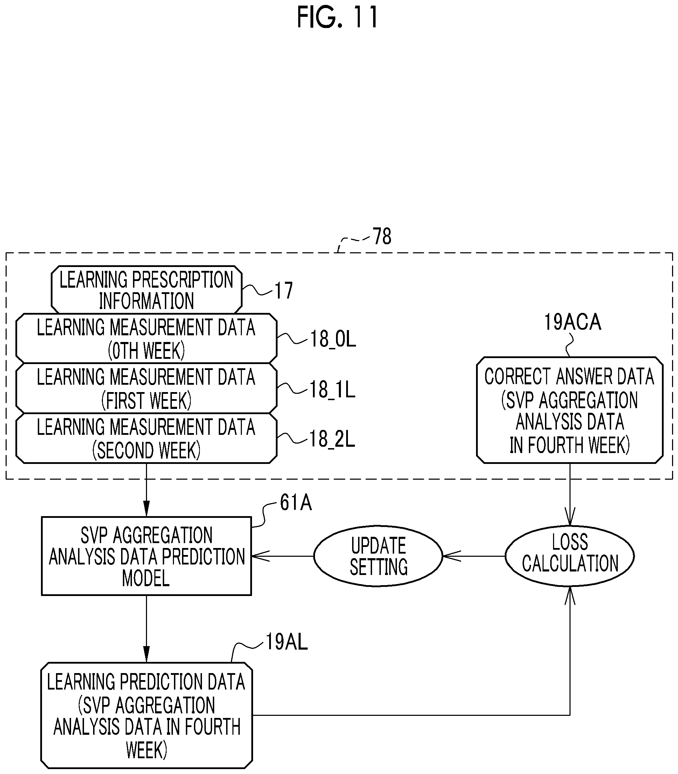

As shown in a table 75 of Fig. 10 as an example, a plurality of sets of learning prescription information 17L, learning measurement data 18_0L, learning measurement data 18_1L, learning measurement data 18_2L, and correct answer data 19ACA are prepared as the learning data 78 (see Fig. 11) of the SVP aggregation analysis data prediction model 61A. The learning prescription information 17L includes the type of additive contained in the preservation solution prepared for learning (hereinafter, referred to as a learning preservation solution) and the hydrogen ion exponent of the learning preservation solution. As in the case of the prescription information 17, the type of the additive is digitized. The learning measurement data 18_0L to 18_2L are data obtained by actually measuring the preservation stability of the learning preservation solution prepared in accordance with the learning prescription information 17L. The learning preservation solution is a preservation solution prepared in the past for various antibodies 37.

The learning measurement data 18_0L includes the SVP aggregation analysis data 40, the DLS analysis data 41, and the SEC analysis data 42 measured after the freezing and thawing test. In addition, the learning measurement data 18_0L includes the SVP aggregation analysis data 40, the DLS analysis data 41, and the SEC analysis data 42 measured after the stirring test. Further, the learning measurement data 18_0L includes the SVP aggregation analysis data 40, the DLS analysis data 41, and the SEC analysis data 42 of the temporal deterioration test. Further, the learning measurement data 18_0L includes the DSC analysis data 43.

The learning measurement data 18_1L and 18_2L include the SVP aggregation analysis data 40, the DLS analysis data 41, and the SEC analysis data 42 of the temporal deterioration test.

The correct answer data 19ACA is data obtained by actually measuring the preservation stability of the learning preservation solution prepared in accordance with the learning prescription information 17L, similarly to the learning measurement data 18_0L to 18_2L. Specifically, the correct answer data 19ACA is the SVP aggregation analysis data 40 actually measured in the fourth week of the stress test.

As illustrated in Fig. 11 as an example, learning data 78 is given in a learning phase of the SVP aggregation analysis data prediction model 61A. The learning data 78 is one of a plurality of sets of the learning prescription information 17L, the learning measurement data 18_0L to 18_2L, and the correct answer data 19ACA shown in the table 75 of Fig. 10. The learning prescription information 17L and the learning measurement data 18_0L to 18_2L in the learning data 78 are input to the SVP aggregation analysis data prediction model 61A. Then, as a result, learning prediction data 19AL is output from the SVP aggregation analysis data prediction model 61A.

The loss calculation of the SVP aggregation analysis data prediction model 61A using the loss function is performed based on the learning prediction data 19AL and the correct answer data 19ACA. Then, the update setting of various coefficients of the SVP aggregation analysis data prediction model 61A is performed in accordance with the result of the loss calculation, and the SVP aggregation analysis data prediction model 61A is updated in accordance with the update setting.

In the learning phase of the SVP aggregation analysis data prediction model 61A, the series of processes of inputting the learning prescription information 17L and the learning measurement data 18_0L to 18_2L to the SVP aggregation analysis data prediction model 61A, outputting the learning prediction data 19AL from the SVP aggregation analysis data prediction model 61A, loss calculation, update setting, and update of the SVP aggregation analysis data prediction model 61A is repeatedly performed while the learning data 78 is exchanged. The repetition of the series of processes is ended in a case where the prediction accuracy of the learning prediction data 19AL with respect to the correct answer data 19ACA reaches a predetermined set level. The SVP aggregation analysis data prediction model 61A in which the prediction accuracy reaches the set level in this manner is stored in the storage 50 as the preservation stability prediction model 61 and is used by the prediction unit 67. The learning may be ended in a case where the series of processes is repeated the set number of times, regardless of the prediction accuracy of the learning prediction data 19AL with respect to the correct answer data 19ACA.

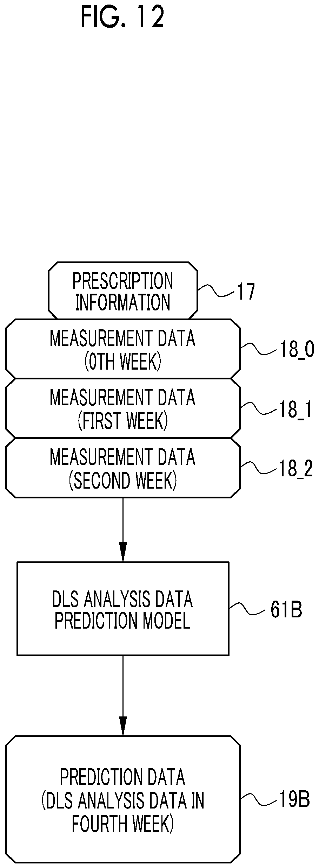

As illustrated in Fig. 12 as an example, the prediction unit 67 inputs a set of the prescription information 17 and the measurement data 18_0 to 18_2 of one candidate preservation solution 35 to the DLS analysis data prediction model 61B. Then, the prediction data 19B is output from the DLS analysis data prediction model 61B. The prediction data 19B is data in which the DLS analysis data 41 in the fourth week of the stress test is predicted. In this case, the DLS analysis data 41 is an example of "first measurement data" according to the technology of the present disclosure. In addition, the SVP aggregation analysis data 40, the SEC analysis data 42, and the DSC analysis data 43 other than the DLS analysis data 41 are examples of "second measurement data" according to the technology of the present disclosure.

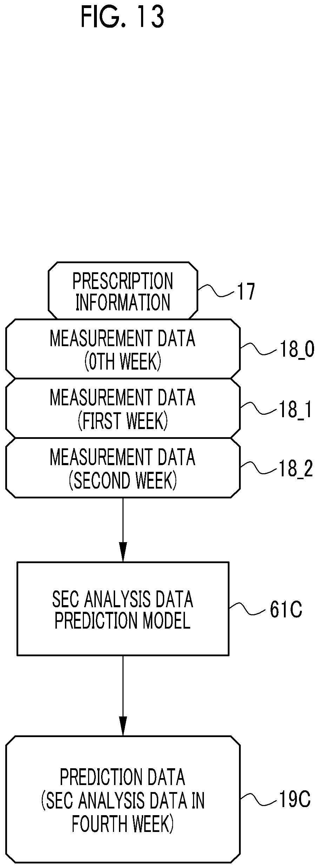

In addition, as illustrated in Fig. 13 as an example, the prediction unit 67 inputs a set of the prescription information 17 and the measurement data 18_0 to 18_2 of one candidate preservation solution 35 to the SEC analysis data prediction model 61C. Then, the prediction data 19C is output from the SEC analysis data prediction model 61C. The prediction data 19C is data in which the SEC analysis data 42 of the fourth week of the stress test is predicted. In this case, the SEC analysis data 42 is an example of "first measurement data" according to the technology of the present disclosure. In addition, the SVP aggregation analysis data 40, the DLS analysis data 41, and the DSC analysis data 43 other than the SEC analysis data 42 are examples of "second measurement data" according to the technology of the present disclosure.

As described above, the SVP aggregation analysis data prediction model 61A, the DLS analysis data prediction model 61B, and the SEC analysis data prediction model 61C constituting the preservation stability prediction model 61 output the prediction data 19A, 19B, and 19C of one type of first measurement data, respectively.

The learning data of the DLS analysis data prediction model 61B is the same as the learning data 78 of the SVP aggregation analysis data prediction model 61A, except that the correct answer data is the DLS analysis data 41 in the fourth week of the stress test. The learning phase of the DLS analysis data prediction model 61B is the same as the learning phase of the SVP aggregation analysis data prediction model 61A, except that the learning prediction data is the DLS analysis data 41 in the fourth week of the stress test. Therefore, illustration and detailed description thereof will be omitted.

Similarly, the learning data of the SEC analysis data prediction model 61C is the same as the learning data 78 of the SVP aggregation analysis data prediction model 61A, except that the correct answer data is the SEC analysis data 42 in the fourth week of the stress test. The learning phase of the SEC analysis data prediction model 61C is the same as the learning phase of the SVP aggregation analysis data prediction model 61A, except that the learning prediction data is the SEC analysis data 42 in the fourth week of the stress test. Therefore, illustration and detailed description thereof will be omitted.

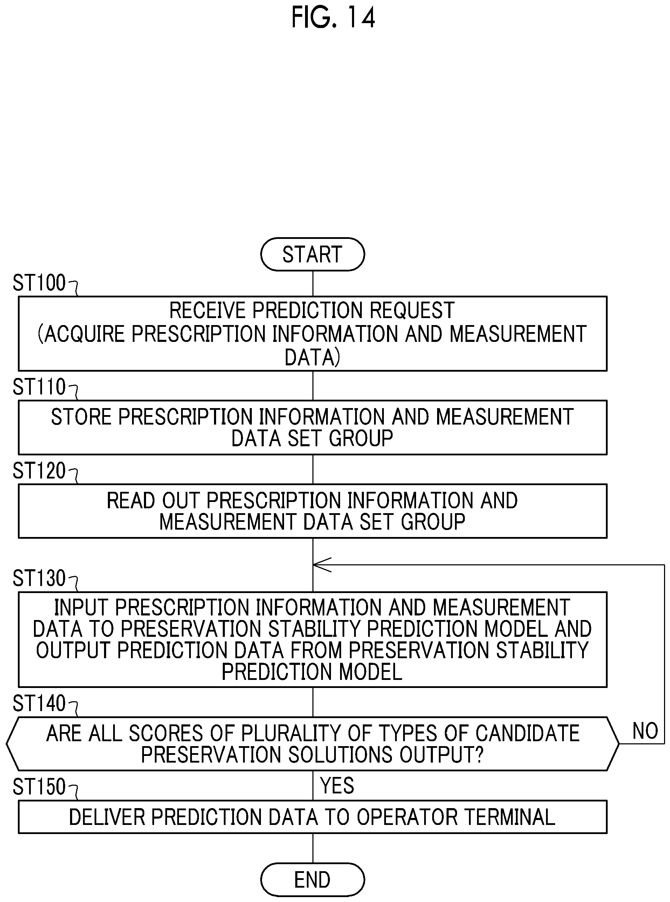

Next, an operation with the configuration described above will be described with reference to the flowchart illustrated in Fig. 14 as an example. First, in a case where the operation program 60 is started in the pharmaceutical support server 10, the CPU 52 of the pharmaceutical support server 10 functions as the receiving unit 65, the RW control unit 66, the prediction unit 67, and the delivery control unit 68 as illustrated in Fig. 7.

The operator inputs a plurality of pieces of desired prescription information 17 and measurement data 18 through the input device 14. Then, the prediction request 15 including the obtained prescription information and measurement data set group 16 is transmitted from the operator terminal 11 to the pharmaceutical support server 10.

In the pharmaceutical support server 10, the prediction request 15 is received by the receiving unit 65. Accordingly, the prescription information and measurement data set group 16, that is, the prescription information 17 and the measurement data 18 are acquired (Step ST100). The prescription information and measurement data set group 16 is output from the receiving unit 65 to the RW control unit 66 and is stored in the storage 50 under the control of the RW control unit 66 (Step ST110).

The RW control unit 66 reads out the prescription information and measurement data set group 16 from the storage 50 (Step ST120). The prescription information and measurement data set group 16 is output from the RW control unit 66 to the prediction unit 67.

In the prediction unit 67, the prescription information 17 and the measurement data 18 of one candidate preservation solution 35 are input to the preservation stability prediction model 61, and the prediction data 19 is output from the preservation stability prediction model 61 (Step ST130). More specifically, as illustrated in Fig. 9, the prescription information 17 and the measurement data 18_0 to 18_2 are input to the SVP aggregation analysis data prediction model 61A, and the prediction data 19A is output from the SVP aggregation analysis data prediction model 61A. In addition, as illustrated in Fig. 12, the prescription information 17 and the measurement data 18_0 to 18_2 are input to the DLS analysis data prediction model 61B, and the prediction data 19B is output from the DLS analysis data prediction model 61B. Further, as illustrated in Fig. 13, the prescription information 17 and the measurement data 18_0 to 18_2 are input to the SEC analysis data prediction model 61C, and the prediction data 19C is output from the SEC analysis data prediction model 61C. The prediction data 19A to 19C are output from the prediction unit 67 to the delivery control unit 68. The process in Step ST130 is repeatedly continued while all the prediction data 19 of the plurality of types of candidate preservation solutions 35 are not output (NO in Step ST140).

In a case where all the prediction data 19 of the plurality of types of candidate preservation solutions 35 are output (YES in Step ST140), the prediction data 19 is delivered to the operator terminal 11, which is the transmission source of the prediction request 15, under the control of the delivery control unit 68 (Step ST 150).

The prediction data 19 is displayed on the display 13 of the operator terminal 11. The operator selects the candidate preservation solution 35 to be adopted as the preservation solution for the biopharmaceutical from the plurality of types of candidate preservation solutions 35 based on the prediction data 19.

As described above, the CPU 52 of the pharmaceutical support server 10 comprises the receiving unit 65 and the prediction unit 67. The receiving unit 65 acquires the prescription information 17 and the measurement data 18 by receiving the prediction request 15. The prescription information 17 is information related to a prescription of the candidate preservation solution 35 which is a candidate for a preservation solution for a biopharmaceutical containing the antibody 37. The measurement data 18 is data actually measured in a test for confirming the preservation stability of the candidate preservation solution 35.

The prediction unit 67 uses the preservation stability prediction model 61 that outputs the prediction data 19 at the future time point of the first measurement data, which is at least one type of the plurality of types of measurement data 18 such as the SVP aggregation analysis data 40, the DLS analysis data 41, the SEC analysis data 42, and the DSC analysis data 43. The prediction unit 67 inputs the prescription information 17, the first measurement data, and at least one type of the second measurement data other than the first measurement data among the plurality of types of measurement data 18 to the preservation stability prediction model 61, and outputs the prediction data 19 from the preservation stability prediction model 61.

For example, the prediction unit 67 inputs the prescription information 17, the SVP aggregation analysis data 40_0 to 40_2 in the 0th to second week of the stress test, the DLS analysis data 41_0 to 41_2 in the 0th to second week of the stress test, the SEC analysis data 42_0 to 42_2 in the 0th to second week of the stress test, and the DSC analysis data 43 to the SVP aggregation analysis data prediction model 61A, and outputs the prediction data 19A, which is the SVP aggregation analysis data 40 in the fourth week, from the SVP aggregation analysis data prediction model 61A. Since not only the first measurement data but also the second measurement data correlated with the first measurement data contribute to the prediction of the prediction data 19, it is possible to improve the prediction accuracy of the preservation stability of the candidate preservation solution 35 as compared with a case where the second measurement data is not input to the preservation stability prediction model 61.

The measurement data 18 is time-series data measured at least at two time points. Therefore, a large amount of data serving as a clue for prediction of the prediction data 19 can be input to the preservation stability prediction model 61, and the prediction accuracy of the prediction data 19 can be further improved.

As illustrated in Fig. 2, the prescription information 17 includes the type 25 of the additive and the hydrogen ion exponent 26. Therefore, it is possible to select a preservation solution in which at least the type 25 of the additive and the hydrogen ion exponent 26 are suitable for the biopharmaceutical.

The measurement data 18 includes data for confirming the preservation stability of the antibody 37 with respect to aggregation, such as the SVP aggregation analysis data 40, the DLS analysis data 41, and the SEC analysis data 42. In addition, the measurement data 18 includes data for confirming the preservation stability of the antibody 37 with respect to the temperature, such as the DSC analysis data 43. Therefore, it is possible to obtain the prediction data 19 reflecting the preservation stabilities of the antibody 37 with respect to both the aggregation and the temperature.

As illustrated in Fig. 3 and the like, the measurement data 18 includes the SVP aggregation analysis data 40, the DSC analysis data 41, the SEC analysis data 42, and the DSC analysis data 43 of the antibody 37 in the candidate preservation solution 35. Therefore, it is possible to obtain the prediction data 19 that more accurately indicates the preservation stability of the candidate preservation solution 35.

As illustrated in Figs. 9, 12, and 13, the preservation stability prediction model 61 outputs the prediction data 19 of one type of the first measurement data. Therefore, the prediction accuracy of the prediction data 19 can be improved as compared with a case where the prediction data 19 of two or more types of the first measurement data is output by one preservation stability prediction model 61.

The biopharmaceutical including the antibody 37 as the protein is called an antibody drug and is widely used not only for the treatment of chronic diseases, such as cancer, diabetes, and rheumatoid arthritis, but also for the treatment of rare diseases such as hemophilia and a Crohn's disease. Therefore, according to the present example in which the protein is the antibody 37, it is possible to further promote the development of antibody drugs widely used for the treatment of various diseases.

The learning of the SVP aggregation analysis data prediction model 61A illustrated in Fig. 11 and the learning of the DLS analysis data prediction model 61B and the SEC analysis data prediction model 61C (not illustrated) may be performed by the pharmaceutical support server 10 or may be performed by another device other than the pharmaceutical support server 10. In addition, the SVP aggregation analysis data prediction model 61A, the DLS analysis data prediction model 61B, and the SEC analysis data prediction model 61C may continue learning even after being stored in the storage 50.

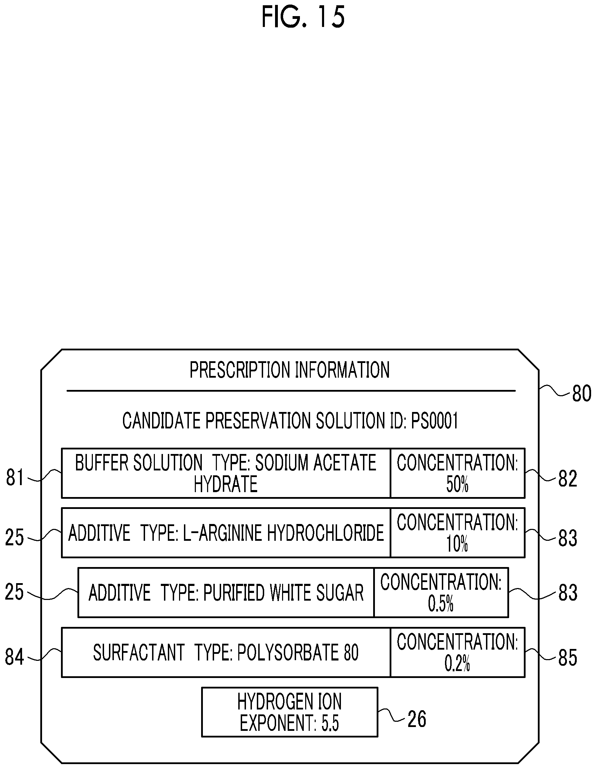

The type 25 of the additive contained in the candidate preservation solution 35 and the hydrogen ion exponent 26 of the candidate preservation solution 35 are exemplified as the prescription information 17, but the present disclosure is not limited thereto. As prescription information 80 illustrated in Fig. 15 as an example, the prescription information may include a type 81 and a concentration 82 of the buffer solution contained in the candidate preservation solution 35, a concentration 83 of the additive, and a type 84 and a concentration 85 of the surfactant. In some cases, each of a plurality of types of buffer solutions, additives, and surfactants is used as can be seen from L-arginine hydrochloride and purified white sugar which are the additives given as an example. In this case, a type and a concentration are registered for each of the plurality of types.

As described above, the prescription information may include at least one of the types 81, 25, and 84 of each of the buffer solution, the additive, and the surfactant contained in the candidate preservation solution 35, the concentrations 82, 83, and 85 of each of the buffer solution, the additive, and the surfactant contained in the candidate preservation solution 35, or the hydrogen ion exponent 26 of the candidate preservation solution 35. Furthermore, a molecular formula of each of the buffer solution, the additive, and the surfactant may be added to the prescription information.

[Second Embodiment]

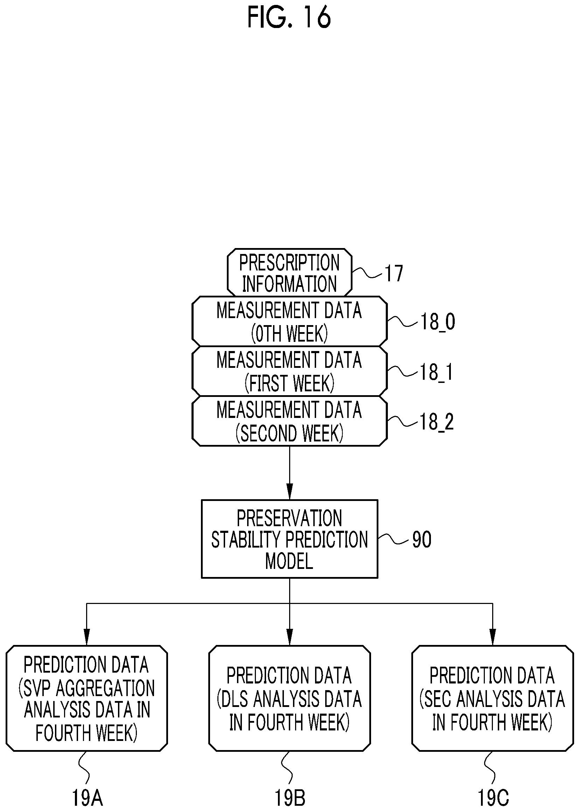

As illustrated in Fig. 16 as an example, the prediction unit 67 inputs a set of the prescription information 17 and the measurement data 18_0 to 18_2 of one candidate preservation solution 35 to the preservation stability prediction model 90. Then, the prediction data 19A, 19B, and 19C are output from the preservation stability prediction model 90. That is, the preservation stability prediction model 90 realizes the functions of the SVP aggregation analysis data prediction model 61A, the DLS analysis data prediction model 61B, and the SEC analysis data prediction model 61C of the first embodiment by one model. The preservation stability prediction model 90 is an example of a "machine learning model" according to the technology of the present disclosure. In addition, in this case, the SVP aggregation analysis data 40, the DLS analysis data 41, and the SEC analysis data 42 are examples of "first measurement data" according to the technology of the present disclosure. In addition, the DSC analysis data 43 is an example of "second measurement data" according to the technology of the present disclosure.

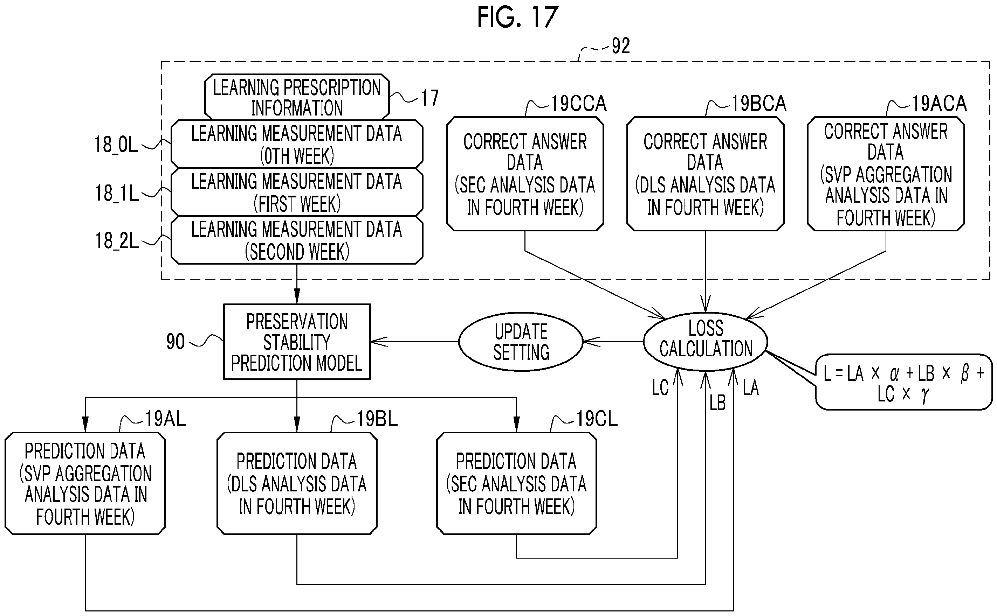

As illustrated in Fig. 17 as an example, learning data 92 is given in the learning phase of the preservation stability prediction model 90. The learning data 92 is a set of the learning prescription information 17L, the learning measurement data 18_0L to 18_2L, and the correct answer data 19ACA, 19BCA, and 19CCA. That is, the learning data 92 is obtained by adding the correct answer data 19BCA and 19CCA to the learning data 78 of the first embodiment. The correct answer data 19BCA and 19CCA are data obtained by actually measuring the preservation stability of the learning preservation solution prepared in accordance with the learning prescription information 17L, similarly to the correct answer data 19ACA. The correct answer data 19BCA is the DLS analysis data 41 in the fourth week of the stress test. Further, the correct answer data 19CCA is the SEC analysis data 42 in the fourth week of the stress test.

The learning prescription information 17L and the learning measurement data 18_0L to 18_2L are input to the preservation stability prediction model 90. Then, as a result, the learning prediction data 19AL, 19BL, and 19CL are output from the preservation stability prediction model 90.

The loss calculation of the preservation stability prediction model 90 using the loss function is performed based on the learning prediction data 19AL and the correct answer data 19ACA. The result of the loss calculation is denoted as a loss LA. In addition, the loss calculation of the preservation stability prediction model 90 using the loss function is performed based on the learning prediction data 19BL and the correct answer data 19BCA. The result of the loss calculation is denoted as a loss LB. Further, the loss calculation of the preservation stability prediction model 90 using the loss function is performed based on the learning prediction data 19CL and the correct answer data 19CCA. The result of the loss calculation is denoted as a loss LC. Then, the update setting of the various coefficients of the preservation stability prediction model 90 is performed in accordance with the total loss L represented in Formula (1), which is the weighted sum of the losses LA to LC, and the preservation stability prediction model 90 is updated in accordance with the update setting. $\alpha$, $\beta$, and $\gamma$ are weights, and $\alpha + \beta + \gamma = 1$.

$$L = LA \times \alpha + LB \times \beta + LC \times \gamma \cdots (1)$$

In the learning phase of the preservation stability prediction model 90, the series of processes of inputting the learning prescription information 17L and the learning measurement data 18_0L to 18_2L to the preservation stability prediction model 90, outputting the learning prediction data 19AL, 19BL, and 19CL from the preservation stability prediction model 90, loss calculation, update setting, and update of the preservation stability prediction model 90 is repeatedly performed while the learning data 92 is exchanged. The repetition of the series of processes is ended in a case where the prediction accuracy of the learning prediction data 19AL with respect to the correct answer data 19ACA, the prediction accuracy of the learning prediction data 19BL with respect to the correct answer data 19BCA, and the prediction accuracy of the learning prediction data 19CL with respect to the correct answer data 19CCA each reaches a predetermined set level. The preservation stability prediction model 90 in which the prediction accuracy reaches the set level in this manner is stored in the storage 50 and is used by the prediction unit 67. The learning may be ended in a case where the series of processes is repeated the set number of times, regardless of the prediction accuracy of the learning prediction data 19AL and the like with respect to the correct answer data 19ACA and the like.

As described above, in the second embodiment, the prediction data 19 of two or more types of the first measurement data is output from one preservation stability prediction model 90. Therefore, as in the first embodiment, it is not necessary to prepare a plurality of machine learning models that output the prediction data 19 of the first measurement data, such as the SVP aggregation analysis data prediction model 61A, the DLS analysis data prediction model 61B, and the SEC analysis data prediction model 61C, and time and effort for the learning of the machine learning model can be saved. In addition, time and effort for the process of outputting the prediction data 19 can also be saved.

[Third Embodiment]

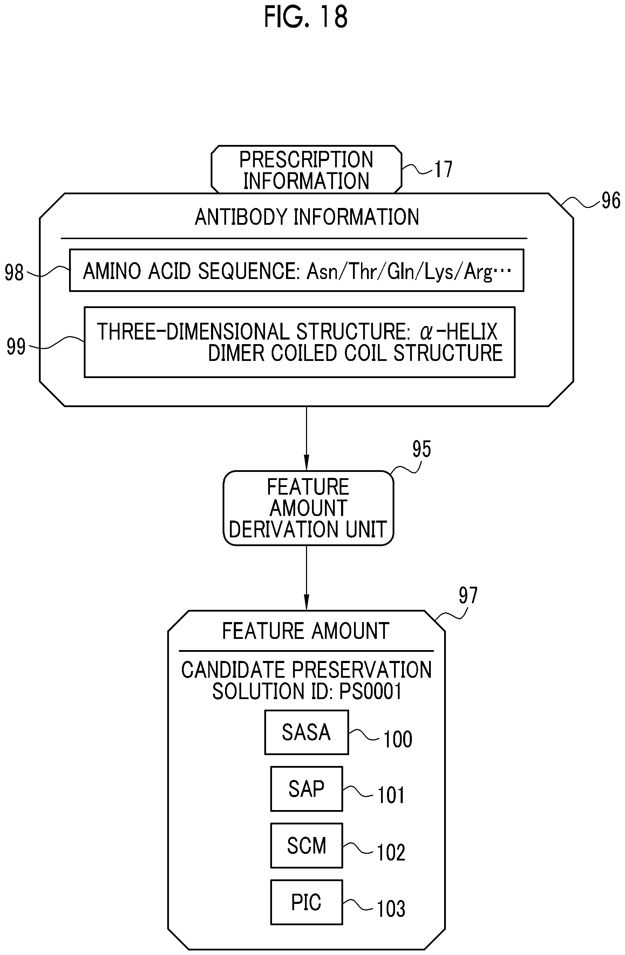

As illustrated in Fig. 18 as an example, the CPU of the pharmaceutical support server according to the third embodiment functions as a feature amount derivation unit 95 in addition to each of the processing units 65 to 68 according to the first embodiment. The feature amount derivation unit 95 derives the feature amount 97 based on the prescription information 17 and the antibody information 96.

The antibody information 96 includes an amino acid sequence 98 of the antibody 37 and a three-dimensional structure 99 of the antibody 37. The antibody information 96 is obtained by analyzing the antibody 37 with a well-known technology such as mass spectrometry, X-ray crystal structure analysis, or electron microscopy. In the amino acid sequence 98, the order of peptide bonds of amino acids constituting the antibody 37, such as asparagine (abbreviated to ASn), glutamine (abbreviated to Glu), and arginine (abbreviated to Arg), is described from an amino terminal to a carboxyl terminal. The amino acid sequence 98 is also referred to as a primary structure. The three-dimensional structure 99 indicates the secondary structure, tertiary structure, and quaternary structure of the amino acids constituting the antibody 37. Examples of the secondary structure include a $\beta$-sheet, a $\beta$-turn, and the like in addition to $\alpha$-helix given. Examples of the tertiary structure include a dimer coiled coil structure and the like. Examples of the quaternary structure include a dimer, a trimer, and a tetramer. Some antibodies 37 do not have the quaternary structure (that is, some antibodies 37 are monomers). In this case, the quaternary structure is not registered as described in the example. The three-dimensional structure 99 can also be obtained by inputting the amino acid sequence 98 to "AlphaFold2" which is a prediction program of a three-dimensional structure of the protein developed by DeepMind Technologies Limited. The antibody information 96 is an example of "protein information" according to the technology of the present disclosure.

The feature amount derivation unit 95 derives, as the feature amount 97, a hydrophobic solvent accessible surface area (hereinafter, abbreviated to SASA) 100 of the antibody 37, a spatial aggregation propensity (hereinafter, abbreviated to SAP) 101 of the antibody 37, and a spatial charge map (hereinafter, abbreviated to SCM) 102 of the antibody 37. As the SASA 100 is larger and the SAP 101 is larger, the stability of the antibody 37 is lower. The SCM 102 indicates the degree of charge of the antibody 37 for each region of the antibody 37. The SASA 100, the SAP 101, and the SCM 102 are derived using a molecular dynamics method. In addition, the SASA 100, the SAP 101, and the SCM 102 are derived based only on the antibody information 96 without referring to the prescription information 17. Therefore, the SASA 100, the SAP 101, and the SCM 102 are common to each candidate preservation solution 35.

Further, the feature amount derivation unit 95 also derives, as the feature amount 97, a preferential interaction coefficient (hereinafter, abbreviated to PIC) 103 described in Non-Patent Document. The PIC 103 indicates, for each region of the antibody 37, the ease of bonding between the antibody 37 and the additive, more specifically, the degree to which the additive covers the surface of the antibody 37, and is an indicator indicating the compatibility between the antibody 37 and the additive. The PIC 103 makes it possible to know the possibility that the aggregation of the antibody 37 causing a reduction in the drug efficacy of the biopharmaceutical will occur. The PIC 103 is derived based on both the prescription information 17 and the antibody information 96 unlike the SASA 100, the SAP 101, and the SCM 102. Therefore, the PIC 103 is different in each candidate preservation solution 35. Therefore, a plurality of feature amounts 97 of each candidate preservation solution 35 have the SASA 100, the SAP 101, and the SCM 102 in common and have different PICs 103. The PIC 103 may be derived using a machine learning model, such as a support vector machine (SVM), instead of the molecular dynamics method. The candidate preservation solution ID is given to the feature amount 97, as in the prescription information 17 and the like.

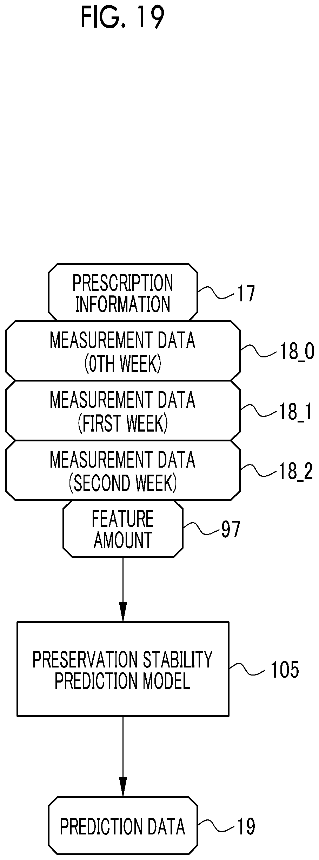

As illustrated in Fig. 19 as an example, in the third embodiment, the prediction unit 67 also inputs the feature amount 97 to the preservation stability prediction model 105 in addition to the prescription information 17 and the measurement data 18_0 to 18 2. Then, the prediction data 19 is output from the preservation stability prediction model 105. The preservation stability prediction model 105 may be any of the SVP aggregation analysis data prediction model 61A, the DLS analysis data prediction model 61B, or the SEC analysis data prediction model 61C of the first embodiment, or may be the preservation stability prediction model 90 of the second embodiment.

As described above, in the third embodiment, the feature amount 97 derived based on the antibody information 96 related to the antibody 37 contained in the biopharmaceutical is also input to the preservation stability prediction model 105. Therefore, it is possible to further improve the prediction accuracy of the prediction data 19.

The feature amount 97 includes the SASA 100, the SAP 101, the SCM 102, and the PIC 103. Therefore, it is possible to obtain the prediction data 19 that more accurately indicates the preservation stability of the candidate preservation solution 35. Further, the feature amount 97 may include at least one of the SASA 100, the SAP 101, the SCM 102, or the PIC 103. Instead of or in addition to the feature amount 97, the antibody information 96 may be digitized and input to the preservation stability prediction model 105.

The measurement data may be any of the SVP aggregation analysis data 40, the DLS analysis data 41, the SEC analysis data 42, or the DSC analysis data 43. As the preservation stability prediction model, a model that outputs, for example, the DSC analysis data 43 in the fourth week as the prediction data 19 in accordance with the input of the prescription information 17 and the measurement data 18_0 to 18_2 may be prepared.

The protein is not limited to the antibody 37 given as an example. Examples of the protein may include cytokine (interferon, interleukin, or the like), hormone (insulin, glucagon, follicle-stimulating hormone, erythropoietin, or the like), a growth factor (insulin-like growth factor (IGF)-1, basic fibroblast growth factor (bFGF), or the like), a blood coagulation factor (a seventh factor, an eighth factor, a ninth factor, or the like), an enzyme (a lysosomal enzyme, a deoxyribonucleic acid (DNA) degrading enzyme, or the like), a fragment crystallizable (Fc) fusion protein, a receptor, albumin, and a protein vaccine. In addition, examples of the antibody include a bispecific antibody, an antibody-drug conjugate, a low-molecular-weight antibody, a sugar-chain-modified antibody, and the like.

The aspect in which the prediction data 19 is provided to be viewed by the operator is not limited to the aspect in which the prediction data 19 is displayed on the display 13. A printed matter of the prediction data 19 may be provided to the operator, or an e-mail to which the prediction data 19 has been attached may be transmitted to a portable terminal of the operator. A score indicating the preservation stability of the candidate preservation solution 35 may be derived based on the prediction data 19 by using a machine learning model or the like. The derived score may be delivered to the operator terminal 11 instead of or in addition to the prediction data 19.

The pharmaceutical support server 10 may be installed in each pharmaceutical facility or may be installed in a data center independent of the pharmaceutical facility. In addition, the operator terminal 11 may be configured to have some or all of the functions of each of the processing units 65 to 68 of the pharmaceutical support server 10.

In each of the above-described embodiments, for example, the following various processors can be used as a hardware structure of processing units executing various processes, such as the receiving unit 65, the RW control unit 66, the prediction unit 67, the delivery control unit 68, and the feature amount derivation unit 95. The various processors include, for example, the CPU 52 which is a general-purpose processor executing software (operation program 60) to function as various processing units as described above, a programmable logic device (PLD), such as a field programmable gate array (FPGA), which is a processor whose circuit configuration can be changed after manufacture, and a dedicated electric circuit, such as an application specific integrated circuit (ASIC), which is a processor having a dedicated circuit configuration designed to execute a specific process.

One processing unit may be configured by one of the various types of processors or may be configured by a combination of two or more processors of the same type or different types (for example, a combination of a plurality of FPGAs and/or a combination of a CPU and an FPGA). Further, a plurality of processing units may be configured by one processor.

As an example of configuring the plurality of processing units with one processor, first, there is an aspect in which one processor is configured by a combination of one or more CPUs and software and the processor functions as the plurality of processing units, as represented by computers such as a client and a server. A second example of the configuration is an aspect in which a processor that implements the functions of the entire system including a plurality of processing units using one integrated circuit (IC) chip is used. A representative example of this aspect is a system on chip (SoC). As described above, the various processing units are configured by using one or more of the above various processors as the hardware structure.

In addition, more specifically, an electric circuit (circuitry) in which circuit elements, such as semiconductor elements, are combined can be used as the hardware structure of the various processors.

It is possible to understand the technology described in the following supplementary notes from the above description.

[Supplementary Note 1]

A pharmaceutical support device comprising:

a processor,
in which the processor is configured to:

acquire prescription information related to a prescription of a candidate preservation solution that is a candidate for a preservation solution for a biopharmaceutical containing a protein;
acquire a plurality of types of measurement data actually measured in a test for confirming preservation stability of the candidate preservation solution;
use a machine learning model that outputs prediction data at a future time point of first measurement data, which is at least one type of the plurality of types of measurement data; and
input the prescription information, the first measurement data, and at least one type of second measurement data other than the first measurement data among the plurality of types of measurement data to the machine learning model and output the prediction data from the machine learning model.

[Supplementary Note 2]

The pharmaceutical support device according to Supplementary Note 1,
in which the measurement data is time-series data measured at least at two time points.

[Supplementary Note 3]

The pharmaceutical support device according to Supplementary Note 1 or 2,
in which the prescription information includes at least one of a type of each of a buffer solution, an additive, or a surfactant contained in the candidate preservation solution, a concentration of each of the buffer solution, the additive, or the surfactant, or a hydrogen ion exponent of the candidate preservation solution.

[Supplementary Note 4]

The pharmaceutical support device according to any one of Supplementary Notes 1 to 3, in which the measurement data includes data for confirming preservation stability of the protein with respect to aggregation and data for confirming preservation stability of the protein with respect to a temperature.

[Supplementary Note 5]

The pharmaceutical support device according to Supplementary Note 4, in which the measurement data is any of aggregation analysis data of a sub-visible particle of the protein in the candidate preservation solution, analysis data of the protein in the candidate preservation solution by a dynamic light scattering method, analysis data of the protein in the candidate preservation solution by size exclusion chromatography, or analysis data of the protein in the candidate preservation solution by differential scanning calorimetry.

[Supplementary Note 6]

The pharmaceutical support device according to any one of Supplementary Notes 1 to 5, in which the machine learning model outputs the prediction data of one type of the first measurement data.

[Supplementary Note 7]

The pharmaceutical support device according to any one of Supplementary Notes 1 to 5, in which the machine learning model outputs the prediction data of two or more types of the first measurement data.

[Supplementary Note 8]

The pharmaceutical support device according to any one of Supplementary Notes 1 to 7, in which the processor is configured to also input a feature amount derived based on protein information related to the protein to the machine learning model.

[Supplementary Note 9]

The pharmaceutical support device according to any one of Supplementary Notes 1 to 8, in which the protein is an antibody.

The above various embodiments and/or various modification examples can be combined as appropriate in the technology of the present disclosure. In addition, it goes without saying that the present disclosure is not limited to each of the above-described embodiments, and various configurations can be adopted without departing from the gist of the present disclosure.

Further, the technology of the present disclosure extends to a storage medium that stores the program non-transitorily, in addition to the program.

The description content and the illustrated content described above are detailed descriptions of portions according to the technology of the present disclosure and are merely an example of the technology of the present disclosure. For example, the above description of the configurations, functions, operations, and effects is the description of examples of the configurations, functions, operations, and effects of portions according to the technology of the present disclosure. Therefore, it is needless to say that unnecessary portions may be deleted or new elements may be added or replaced in the above description content and illustrated content without departing from the gist of the technology of the present disclosure. In order to avoid complication and facilitate understanding of the portion according to the technology of the present disclosure, the description related to common general knowledge not requiring special description in order to implement the technology of the present disclosure is omitted in the above description content and illustrated content.

In the present specification, "A and/or B" is synonymous with "at least one of A or B". That is, "A and/or B" may mean only A, only B, or a combination of A and B. Further, in the present specification, the same concept as "A and/or B" is also applied to a case where three or more matters are linked and expressed by "and/or".

All documents, patent applications, and technical standards described in the present specification are incorporated in the present specification by reference to the same extent as in a case where each of the documents, patent applications, and technical standards are specifically and individually indicated to be incorporated by reference.

**Claims**

1. A pharmaceutical support device comprising:

   a processor,
   wherein the processor is configured to:

      acquire prescription information related to a prescription of a candidate preservation solution that is a candidate for a preservation solution for a biopharmaceutical containing a protein;
      acquire a plurality of types of measurement data actually measured in a test for confirming preservation stability of the candidate preservation solution;
      use a machine learning model that outputs prediction data at a future time point of first measurement data, which is at least one type of the plurality of types of measurement data; and

input the prescription information, the first measurement data, and at least one type of second measurement data other than the first measurement data among the plurality of types of measurement data to the machine learning model and output the prediction data from the machine learning model.

2. The pharmaceutical support device according to claim 1,
wherein the measurement data is time-series data measured at least at two time points.

3. The pharmaceutical support device according to claim 1,
wherein the prescription information includes at least one of a type of each of a buffer solution, an additive, or a surfactant contained in the candidate preservation solution, a concentration of each of the buffer solution, the additive, or the surfactant, or a hydrogen ion exponent of the candidate preservation solution.

4. The pharmaceutical support device according to claim 1,
wherein the measurement data includes data for confirming preservation stability of the protein with respect to aggregation and data for confirming preservation stability of the protein with respect to a temperature.

5. The pharmaceutical support device according to claim 4,
wherein the measurement data is any of aggregation analysis data of a sub-visible particle of the protein in the candidate preservation solution, analysis data of the protein in the candidate preservation solution by a dynamic light scattering method, analysis data of the protein in the candidate preservation solution by size exclusion chromatography, or analysis data of the protein in the candidate preservation solution by differential scanning calorimetry.

6. The pharmaceutical support device according to claim 1,
wherein the machine learning model outputs the prediction data of one type of the first measurement data.

7. The pharmaceutical support device according to claim 1,
wherein the machine learning model outputs the prediction data of two or more types of the first measurement data.

8. The pharmaceutical support device according to claim 1,
wherein the processor is configured to also input a feature amount derived based on protein information related to the protein to the machine learning model.

9. The pharmaceutical support device according to claim 1,
wherein the protein is an antibody.

10. An operation method of a pharmaceutical support device, comprising:

acquiring prescription information related to a prescription of a candidate preservation solution that is a candidate for a preservation solution for a biopharmaceutical containing a protein;
acquiring a plurality of types of measurement data actually measured in a test for confirming preservation stability of the candidate preservation solution;
using a machine learning model that outputs prediction data at a future time point of first measurement data, which is at least one type of the plurality of types of measurement data; and
inputting the prescription information, the first measurement data, and at least one type of second measurement data other than the first measurement data among the plurality of types of measurement data to the machine learning model and outputting the prediction data from the machine learning model.

11. An operation program of a pharmaceutical support device causing a computer to execute a process comprising:

acquiring prescription information related to a prescription of a candidate preservation solution that is a candidate for a preservation solution for a biopharmaceutical containing a protein;
acquiring a plurality of types of measurement data actually measured in a test for confirming preservation stability of the candidate preservation solution;
using a machine learning model that outputs prediction data at a future time point of first measurement data, which is at least one type of the plurality of types of measurement data; and
inputting the prescription information, the first measurement data, and at least one type of second measurement data other than the first measurement data among the plurality of types of measurement data to the machine learning model and outputting the prediction data from the machine learning model.

FIG. 1

EP 4 506 948 A1

## FIG. 2

PRESCRIPTION INFORMATION — 17

CANDIDATE PRESERVATION SOLUTION ID: PS0001

ADDITIVE TYPE: L-ARGININE HYDROCHLORIDE — 25

HYDROGEN ION EXPONENT: 5.5 — 26

37

ADDITION

36

35

PREPARATION

STRESS TEST (FOR TWO WEEKS)
· FREEZING AND THAEWING TEST
· STIRRING TEST
· TEMPORAL DETERIORATION TEST

38

18_0 — MEASUREMENT DATA (0TH WEEK)

18_1 — MEASUREMENT DATA (FIRST WEEK)

18_2 — MEASUREMENT DATA (SECOND WEEK)

EP 4 506 948 A1

## FIG. 3

**18_0** — MEASUREMENT DATA (0TH WEEK)

CANDIDATE PRESERVATION SOLUTION ID: PS0001

DATA FOR CONFIRMING PRESERVATION STABILITY OF ANTIBODY WITH RESPECT TO AGGREGATION

**40_0A** — SVP AGGREGATION ANALYSIS DATA (FREEZING AND THAWING TEST)

**40_0B** — SVP AGGREGATION ANALYSIS DATA (STIRRING TEST)

**41_0A** — DLS ANALYSIS DATA (FREEZING AND THAWING TEST)

**41_0B** — DLS ANALYSIS DATA (STIRRING TEST)

**42_0A** — SEC ANALYSIS DATA (FREEZING AND THAWING TEST)

**42_0B** — SEC ANALYSIS DATA (STIRRING TEST)

**40_0C** — SVP AGGREGATION ANALYSIS DATA (TEMPORAL DETERIORATION TEST)

**41_0C** — DLS ANALYSIS DATA (TEMPORAL DETERIORATION TEST)

**42_0C** — SEC ANALYSIS DATA (TEMPORAL DETERIORATION TEST)

**43** — DSC ANALYSIS DATA

DATA FOR CONFIRMING PRESERVATION STABILITY OF ANTIBODY WITH RESPECT TO TEMPERATURE

EP 4 506 948 A1

## FIG. 4

18_1

MEASUREMENT DATA (FIRST WEEK)

CANDIDATE PRESERVATION
SOLUTION ID: PS0001

DATA FOR CONFIRMING PRESERVATION
STABILITY OF ANTIBODY WITH RESPECT
TO AGGREGATION

SVP AGGREGATION ANALYSIS DATA
(TEMPORAL DETERIORATION TEST) — 40_1C

DLS ANALYSIS DATA
(TEMPORAL DETERIORATION TEST) — 41_1C

SEC ANALYSIS DATA
(TEMPORAL DETERIORATION TEST) — 42_1C

## FIG. 5

18_2

MEASUREMENT DATA (SECOND WEEK)

CANDIDATE PRESERVATION
SOLUTION ID: PS0001

DATA FOR CONFIRMING PRESERVATION
STABILITY OF ANTIBODY WITH RESPECT
TO AGGREGATION

SVP AGGREGATION ANALYSIS DATA
(TEMPORAL DETERIORATION TEST) — 40_2C

DLS ANALYSIS DATA
(TEMPORAL DETERIORATION TEST) — 41_2C

SEC ANALYSIS DATA
(TEMPORAL DETERIORATION TEST) — 42_2C

# FIG. 6

# FIG. 7

## FIG. 8

61

**PRESERVATION STABILITY PREDICTION MODEL**

| SVP AGGREGATION ANALYSIS DATA PREDICTION MODEL | DLS ANALYSIS DATA PREDICTION MODEL | SEC ANALYSIS DATA PREDICTION MODEL |

61A          61B          61C

## FIG. 9

PRESCRIPTION INFORMATION ~17

MEASUREMENT DATA (0TH WEEK) ~18_0

MEASUREMENT DATA (FIRST WEEK) ~18_1

MEASUREMENT DATA (SECOND WEEK) ~18_2

↓

SVP AGGREGATION ANALYSIS DATA PREDICTION MODEL ~61A

↓

PREDICTION DATA (SVP AGGREGATION ANALYSIS DATA IN FOURTH WEEK) ~19A

## FIG. 10

| LEARNING PRESCRIPTION INFORMATION | | LEARNING MEASUREMENT DATA (0TH WEEK) | | | | | | | | | | LEARNING MEASUREMENT DATA (FIRST WEEK) | | | LEARNING MEASUREMENT DATA (SECOND WEEK) | | | CORRECT ANSWER DATA (SVP AGGREGATION ANALYSIS DATA IN FOURTH WEEK) |
|---|---|---|---|---|---|---|---|---|---|---|---|---|---|---|---|---|---|---|
| | | FREEZING AND THAWING TEST | | | STIRRING TEST | | | TEMPORAL DETERIORATION TEST | | | DSC | TEMPORAL DETERIORATION TEST | | | TEMPORAL DETERIORATION TEST | | | |
| TYPE OF ADDITIVE | HYDROGEN ION EXPONENT | SVP | DLS | SEC | SVP | DLS | SEC | SVP | DLS | SEC | | SVP | DLS | SEC | SVP | DLS | SEC | |
| L-ARGININE HYDROCHLORIDE | 6.0 | 16900 | 6.5 | 94.1 | 11174 | 6.6 | 93.7 | 10208 | 6.7 | 92.8 | 50.9 | 11200 | 6.9 | 93.5 | 12100 | 7.1 | 94.4 | 28095 |
| L-ARGININE HYDROCHLORIDE | 6.5 | 12200 | 6.7 | 88.6 | 14855 | 6.8 | 89.1 | 11223 | 6.7 | 89.5 | 53.6 | 12453 | 7.0 | 91.2 | 13650 | 7.2 | 92.2 | 29043 |
| L-ARGININE HYDROCHLORIDE | 7.0 | 14024 | 5.8 | 84.5 | 12656 | 6.1 | 85.6 | 14825 | 5.6 | 88.2 | 58.4 | 15212 | 5.8 | 90.4 | 16715 | 6.0 | 91.8 | 32094 |
| L-ARGININE HYDROCHLORIDE | 7.5 | 9000 | 4.9 | 69.2 | 11021 | 5.1 | 72.5 | 10032 | 4.8 | 72.4 | 55.8 | 11298 | 4.9 | 80.1 | 13026 | 5.2 | 84.6 | 24850 |
| L-HISTIDINE HYDROCHLORIDE | 6.0 | 18092 | 5.1 | 98.4 | 14800 | 5.8 | 92.6 | 19402 | 5.0 | 96.2 | 50.4 | 20026 | 5.1 | 97.4 | 22009 | 5.4 | 98.2 | 34652 |
| L-HISTIDINE HYDROCHLORIDE | 6.5 | 17908 | 6.3 | 79.7 | 16385 | 6.4 | 82.3 | 14658 | 6.1 | 83.9 | 48.1 | 18212 | 6.3 | 88.5 | 19248 | 6.5 | 89.7 | 38821 |
| L-HISTIDINE HYDROCHLORIDE | 7.0 | 15642 | 6.5 | 64.4 | 17124 | 6.7 | 68.2 | 18842 | 6.3 | 72.7 | 52.4 | 19020 | 6.5 | 79.6 | 20032 | 6.7 | 82.4 | 27952 |

⋮

EP 4 506 948 A1

# FIG. 11

LEARNING PRESCRIPTION INFORMATION ~17

LEARNING MEASUREMENT DATA (0TH WEEK) ~18_0L

LEARNING MEASUREMENT DATA (FIRST WEEK) ~18_1L

LEARNING MEASUREMENT DATA (SECOND WEEK) ~18_2L

~78

19ACA

CORRECT ANSWER DATA (SVP AGGREGATION ANALYSIS DATA IN FOURTH WEEK)

SVP AGGREGATION ANALYSIS DATA PREDICTION MODEL ~61A

UPDATE SETTING

LOSS CALCULATION

LEARNING PREDICTION DATA (SVP AGGREGATION ANALYSIS DATA IN FOURTH WEEK) ~19AL

# FIG. 12

PRESCRIPTION INFORMATION —— 17

MEASUREMENT DATA (0TH WEEK) —— 18_0

MEASUREMENT DATA (FIRST WEEK) —— 18_1

MEASUREMENT DATA (SECOND WEEK) —— 18_2

DLS ANALYSIS DATA PREDICTION MODEL —— 61B

PREDICTION DATA (DLS ANALYSIS DATA IN FOURTH WEEK) —— 19B

# FIG. 13

PRESCRIPTION INFORMATION —17

MEASUREMENT DATA (0TH WEEK) —18_0

MEASUREMENT DATA (FIRST WEEK) —18_1

MEASUREMENT DATA (SECOND WEEK) —18_2

↓

SEC ANALYSIS DATA PREDICTION MODEL —61C

↓

PREDICTION DATA (SEC ANALYSIS DATA IN FOURTH WEEK) —19C

# FIG. 14

```
                         ( START )
                             │
ST100                        │
┌────────────────────────────────────────────────────────┐
│              RECEIVE PREDICTION REQUEST                  │
│  (ACQUIRE PRESCRIPTION INFORMATION AND MEASUREMENT       │
│                       DATA)                              │
└────────────────────────────────────────────────────────┘
ST110                        │
┌────────────────────────────────────────────────────────┐
│   STORE PRESCRIPTION INFORMATION AND MEASUREMENT         │
│                  DATA SET GROUP                          │
└────────────────────────────────────────────────────────┘
ST120                        │
┌────────────────────────────────────────────────────────┐
│       READ OUT PRESCRIPTION INFORMATION AND             │
│            MEASUREMENT DATA SET GROUP                    │
└────────────────────────────────────────────────────────┘
                             │
ST130                        │
┌────────────────────────────────────────────────────────┐
│     INPUT PRESCRIPTION INFORMATION AND MEASUREMENT      │
│   DATA TO PRESERVATION STABILITY PREDICTION MODEL AND    │
│   OUTPUT PREDICTION DATA FROM PRESERVATION STABILITY     │
│                  PREDICTION MODEL                        │
└────────────────────────────────────────────────────────┘
ST140                        │
  ARE ALL SCORES OF PLURALITY OF TYPES OF CANDIDATE       NO
      PRESERVATION SOLUTIONS OUTPUT?
ST150                     │ YES
┌────────────────────────────────────────────────────────┐
│      DELIVER PREDICTION DATA TO OPERATOR TERMINAL        │
└────────────────────────────────────────────────────────┘
                             │
                         ( END )
```

# FIG. 15

PRESCRIPTION INFORMATION

CANDIDATE PRESERVATION SOLUTION ID: PS0001

| | |
|---|---|
| BUFFER SOLUTION  TYPE: SODIUM ACETATE HYDRATE | CONCENTRATION: 50% |
| ADDITIVE  TYPE: L-ARGININE HYDROCHLORIDE | CONCENTRATION: 10% |
| ADDITIVE  TYPE: PURIFIED WHITE SUGAR | CONCENTRATION: 0.5% |
| SURFACTANT  TYPE: POLYSORBATE 80 | CONCENTRATION: 0.2% |

HYDROGEN ION EXPONENT: 5.5

~80
81~
~82
25~
~83
25~
~83
84~
~85
~26

# FIG. 16

PRESCRIPTION INFORMATION ~17

MEASUREMENT DATA (0TH WEEK) ~18_0

MEASUREMENT DATA (FIRST WEEK) ~18_1

MEASUREMENT DATA (SECOND WEEK) ~18_2

PRESERVATION STABILITY PREDICTION MODEL ~90

PREDICTION DATA (SVP AGGREGATION ANALYSIS DATA IN FOURTH WEEK)

19A

PREDICTION DATA (DLS ANALYSIS DATA IN FOURTH WEEK)

19B

PREDICTION DATA (SEC ANALYSIS DATA IN FOURTH WEEK)

19C

## FIG. 17

**92**

**LEARNING PRESCRIPTION INFORMATION** ~17

**18_0L** — LEARNING MEASUREMENT DATA (0TH WEEK)

**18_1L** — LEARNING MEASUREMENT DATA (FIRST WEEK)

**18_2L** — LEARNING MEASUREMENT DATA (SECOND WEEK)

~19CCA CORRECT ANSWER DATA (SEC ANALYSIS DATA IN FOURTH WEEK)

~19BCA CORRECT ANSWER DATA (DLS ANALYSIS DATA IN FOURTH WEEK)

~19ACA CORRECT ANSWER DATA (SVP AGGREGATION ANALYSIS DATA IN FOURTH WEEK)

**90** — PRESERVATION STABILITY PREDICTION MODEL

UPDATE SETTING

LOSS CALCULATION

$L = LA \times \alpha + LB \times \beta + LC \times \gamma$

LC    LB    LA

~19AL PREDICTION DATA (SVP AGGREGATION ANALYSIS DATA IN FOURTH WEEK)

~19BL PREDICTION DATA (DLS ANALYSIS DATA IN FOURTH WEEK)

~19CL PREDICTION DATA (SEC ANALYSIS DATA IN FOURTH WEEK)

EP 4 506 948 A1

# FIG. 18

PRESCRIPTION INFORMATION ~17

ANTIBODY INFORMATION ~96

AMINO ACID SEQUENCE: Asn/Thr/Gln/Lys/Arg··· ~98

THREE-DIMENSIONAL STRUCTURE: $\alpha$-HELIX DIMER COILED COIL STRUCTURE ~99

FEATURE AMOUNT DERIVATION UNIT ~95

FEATURE AMOUNT ~97

CANDIDATE PRESERVATION SOLUTION ID: PS0001

SASA ~100

SAP ~101

SCM ~102

PIC ~103

# FIG. 19

PRESCRIPTION
INFORMATION ～17

MEASUREMENT DATA
(0TH WEEK) ～18_0

MEASUREMENT DATA
(FIRST WEEK) ～18_1

MEASUREMENT DATA
(SECOND WEEK) ～18_2

FEATURE
AMOUNT ～97

PRESERVATION STABILITY
PREDICTION MODEL ～105

PREDICTION DATA ～19

## INTERNATIONAL SEARCH REPORT

International application No.

**PCT/JP2023/014063**

### A. CLASSIFICATION OF SUBJECT MATTER

*G16B 40/20*(2019.01)i; *C07K 16/00*(2006.01)i; *C12N 15/13*(2006.01)i; *G01N 33/15*(2006.01)i; *G16H 20/10*(2018.01)i
FI:    G16B40/20; G01N33/15 Z; C07K16/00; C12N15/13; G16H20/10

According to International Patent Classification (IPC) or to both national classification and IPC

### B. FIELDS SEARCHED

Minimum documentation searched (classification system followed by classification symbols)

G16B 5/00-99/00; C07K16/00; C12N15/13; G01N33/15; G16H20/10

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | 松岡常吉, 冨田峻介, 平野篤, タンパク質熱凝集を抑制する化合物, [online], 2008, pp. 1-7, [retrieved on 9 May 2023], Internet: <URL:http://www.pssj.jp/archives/files/articles/041.pdf>, non-official translation (MATSUOKA, Tsunekichi, TOMITA, Shunsuke, HIRANO, Atsushi. Compounds that Inhibit Protein Thermal Aggregation.)<br>    entire text, all drawings | 1-11 |
| A | WO 2021/041384 A1 (AMGEN INC.) 04 March 2021 (2021-03-04)<br>    entire text, all drawings | 1-11 |
| A | CLOUTIER, Thereksa K et al. Machine Learning Models of Antibody-Excipient Preferential Interactions for Use in Computational Formulation Design. Molecular Pharmaceutics. 14 August 2020, vol. 17, issue 9, pp. 3589-3599<br>    entire text, all drawings | 1-11 |

☐ Further documents are listed in the continuation of Box C.　　☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **09 May 2023** | **16 May 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

INTERNATIONAL SEARCH REPORT

Information on patent family members

International application No.

**PCT/JP2023/014063**

| Patent document cited in search report | Publication date (day/month/year) | Patent family member(s) | Publication date (day/month/year) |
|---|---|---|---|
| WO 2021/041384 A1 | 04 March 2021 | US 2022/0293223 A1<br>entire text, all drawings<br>EP 4022622 A1<br>AU 2020340312 A1<br>CA 3141708 A1<br>IL 288527 A<br>SG 11202112890V A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **THERESA K. CLOUTIER**. Machine Learning Models of Antibody-Excipient Preferential Interactions for Use in Computational Formulation Design. *Mol. Pharmaceutics*, 2020, vol. 17 (9), 3589-3599